# EUROPEAN PATENT APPLICATION

(11) **EP 3 575 310 A1**
(43) Date of publication of application: **04.12.2019**
(21) Application number: 19460029.2
(22) Date of filing: 28.05.2019
(51) Int. Cl.: C07J 63/00, A61K 31/575, A61P 31/18

(54) **PHOSPHONATE DERIVATES OF BETULIN COMPOUNDS WITH ANTIVIRAL ACTIVITY, METHOD FOR THEIR PREPARATION AND THEIR APPLICATION**

(30) Priority: 28.05.2018 PL 42575518
(71) Applicant: Uniwersystet Slaski w Katowicach, 40-007 Katowice (PL); Narodowy Instytut Leków, 00-725 Warszawa (PL); Slaski Uniwerytet Medyczny, 40-055 Katowice (PL)
(72) Inventor: Boryczka, Stanislaw, 41-209 Sosnowiec (PL); Chrobak, Elwira, 40-877 Katowice (PL); Bebenek, Ewa, 41-300 Dabrowa Górnicza (PL); Kadela-Tomanek, Monika, 40-018 Katowice (PL); Dabrowska, Aleksandra, 26-600 Radom (PL); Chilmonczyk, Zdzislaw, 01-904 Warszawa (PL); Wiktorska, Katarzyna, 02-791 Warszawa (PL); Milczarek, Malgorzata, 02-798 Warszawa (PL); Jastrzebska, Maria, 40-561 Katowice (PL)

(57) **Abstract**

The invention relates to the phosphonate derivatives of the 3-carboxyacylbetulinic acid with antiviral activity, of formula 1A or of formula 1B, wherein:
R is an alkyl group (C₁-C₄),
R₁ is an alkyl group (C₁-C₄),
R₂ is a hydroxyl or carbonyl or acetyloxy or carboxyacyloxy group,
R₃ is an acetyloxymethyl or hydroxymethyl or carboxyl group. 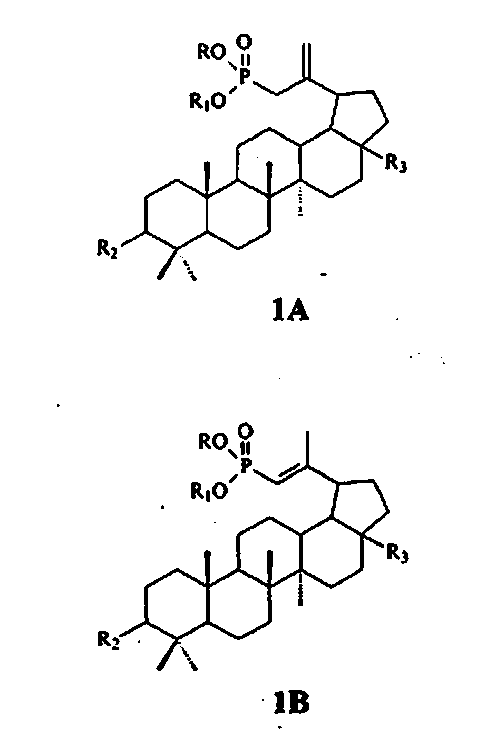 The invention also relates to a method of preparing the derivatives of formula 1A, where there are following steps: (a) compound of formula 2A, wherein: R is an alkyl group (C₁-C₄), and R₁ is an alkyl group (C₁-C₄), is subjected to alkaline hydrolysis in the presence of an aqueous solution of a metal hydroxide, in an organic solvent or in a mixture of organic solvents, resulting in 30-phosphonate of 3-acetylbetulin; (b) the product of step (a) is reacted with the Jones reagent taken in an amount of 1 to 4 ml per 1 mmol of the product of step (a), in at least 10 ml of an organic solvent per 1 mmol of the product of step (a), resulting in 30-phosphonate of 3-acetylbetulinic acid; (c) the product of step (b) is subjected to alkaline hydrolysis in the presence of an aqueous solution of a metal hydroxide in an organic solvent, resulting in 30-phosphonate of betulinic acid; (d) the product of step (c) is reacted with a dicarboxylic acid anhydride or with a dicarboxylic acid, at a molar ratio of between 1:1 to 1:10, for at least 30 minutes.
The invention also relates to a method of preparing the derivatives of formula 1B, where there are following steps: (a) compound of formula 2B, wherein R is an alkyl group (C₁-C₄), and R₁ is an alkyl group (C₁-C₄), is reacted with the Jones reagent taken in an amount of 2 to 6 ml per 1 mmol of the compound of formula 2B, resulting in 29-phosphonate of betulonic acid; (b) the product of step (a) is reduced by sodium or lithium borohydride, resulting in 29-phosphonate of betulinic acid; (c) the product of step (b) is reacted with a dicarboxylic acid anhydride or with a dicarboxylic acid, at a molar ratio of between 1:2.5 to 1:10, for at least 30 minutes.
The invention also relates to the use of phosphonate derivatives of 3-carboxyacylbetulinic acid with antiviral activity, of formula 1A or 1B (wherein: R is an alkyl group (C₁-C₄), R₁ is an alkyl group (C₁-C₄), R₂ is a hydroxyl or carbonyl or acetyloxy or carboxyacyloxy group, R₃ is an acetyloxymethyl or hydroxymethyl or carboxyl group) in the manufacture of pharmaceutical agents for suppressing HIV-1 replication.

## Description

The invention relates to phosphonate derivatives of the 3-carboxyacylbetulinic acid having antiviral activity, method for their preparation and their use in the manufacture of pharmaceutical agents for suppressing HIV-1 replication.

AIDS is a disease that is rapidly spreading among many populations, and due to its enormous extent it has been called a global epidemic. It is caused by HIV (Human Immunodeficiency Virus), a member of the retrovirus family that attacks cells of the immune system, destroying them or impairing their functions. This gradually reduces the ability of the host's to fight infections. According to the Polish National AIDS Centre, since 1985, that is from the beginning of the outbreak of HIV epidemic in Poland, over 22 thousand infections have been reported, resulting in approximately 3,500 people developing AIDS. According to WHO, at the end of 2016 there were 36.7 million people infected with HIV around the world. Intensive research aimed at developing new therapies and implemented preventive programmes have reduced the transmission of the virus. According to the UNAIDS (United Nations Programme on HIV/AIDS), since 2010 the annual number of new infections has decreased by 18% to 1.8 million.

In the case of antiviral drugs used in AIDS monotherapy, such as AZT (azidothymidine), rapid development of resistance to the drug has been observed. It was found that the virus mutates rapidly, which results in the ineffectiveness of treatment based on a single preparation, and promotes the development of drug-resistant strains. Currently, HAART (Highly Active Antiretroviral Therapy) is being used in the fight against this virus, which involves the combination of at least three antiretroviral drugs, e.g. a combination of nucleoside reverse transcriptase inhibitors with a protease inhibitor or with a non-nucleoside reverse transcriptase inhibitor. The increase in the number of available medications was followed by the emergence of interactions between them, which excluded simultaneous use of certain drugs in a single therapeutic regimen. That is why the search for new substances with antiviral activity is still an important issue. These new substances should show different mechanisms of action than currently used therapeutics, which would allow them to be used in a more convenient treatment regimen.

One important method of obtaining new therapeutic agents is the modification of existing substances of known biological activity in order to optimize their properties. Such substances include compounds obtained from natural raw materials, among which is the lupane-type pentacyclic triterpene - betulin (lup-20(29)-ene-3*β*,28-diol), as well as its oxidation product, betulinic acid ((3*β*)-3-hydroxy-lup-20(29)-en-28-oic acid).

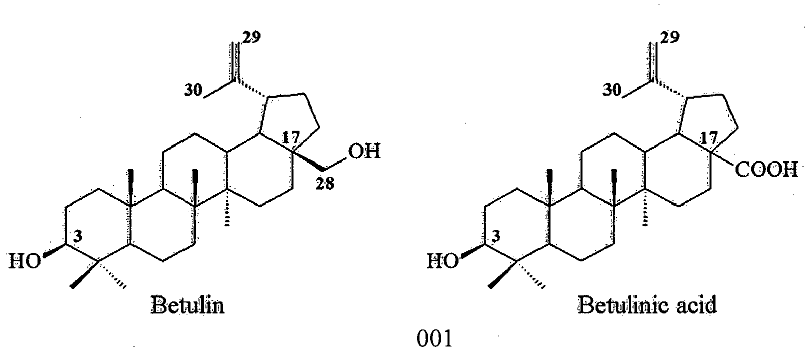

These compounds, as well as their derivatives, exhibit a broad spectrum of biological activity, including antitumour, antiviral, antimalaric, antibacterial, anti-inflammatory and hepatoprotective activities.

In 1994, for the first time, betulin derivatives inhibiting the HIV-1 replication were described. These were betulinic acid (EC₅₀ = 1.6 µM, TI = 9.3) and dihydrobetulinic acid (EC₅₀ = 0.9 µM, TI = 14) (T. Fujioka, Y. "Betulinic acid and platanic acid as anti-HIV principles from Syzigium claviflorum, and the anti-HIV activity of structurally related triterpenoids", J. Nat. Prod., 1994, 57, 243-247). However, it was found, that betulin, betulinic acid and betulonic acid demonstrate weak activity against HIV-1 (H-J. Zhang et al., "Natural anti-HIV agents. Part IV. Anti-HIV constituents from Vatica cinerea", J. Nat. Prod., 2003, 66, 263-268).

Betulinic acid derivatives represent a promising class of compounds active against the HIV virus. Also in 1994, Mayaux et al. from the Rhône-Poulenc Rorer Pharmaceuticals, described the amide derivatives of betulinic acid, designated as RP 70034, RPR 103611, and derivatives of the 30-hydroxybetulinic acid, designated as RP 72046, which were blocking the HIV entry into the cell (J.F. Mayaux et al., "Triterpene derivatives that block entry of human immunodeficiency virus type 1 into cells", Proc. Natl. Acad. Sci. USA, 1994, 91, 3564-3568). Studies of primary amides of betulinic acid with a terminal carboxylic group of various alkyl chain lengths, as well as products of their condensation with *α*, *β* or *γ*-amino acids (introduction of an additional amide group in the side chain) confirmed that the most active compound is the one designated as RPR 103611 and its diastereoisomer IC 9564 (Z. Dang et al., "Synthesis of betulinic acid derivatives as entry inhibitors against HIV-1 and bevirimat-resistant HIV-1 variants", Bioorg. Med. Chem. Lett., 2012, 22, 5190-5194; F. Soler et al., "Betulinic acid derivatives: A new class of specific inhibitors of human immunodeficiency virus type 1 entry", J. Med. Chem., 1996, 39, 1069-1083, US Pat. 1995/005468888A, "Derivatives of 3-*O*-(3',3'-dimethylsuccinyl)betulinic acid").

The greatest hope for a breakthrough in HIV/AIDS therapy was raised by the 3-*O*-(3',3'-dimethylsuccinyl)betulinic acid, synthesized by Kuo-Hsiung Lee's team, which demonstrated the EC₅₀ = 0.0035 µM and TI = 20,000 in relation to the HIV-1 infected H9 lymphocytes (F. Hashimoto et al., "Anti-AIDS agents-XXVII. Synthesis and anti-HIV activity of betulinic acid and dihydrobetulinic acid derivatives", Bioorg. Med. Chem., 1997, 5, 2133-2143, US Pat. 1997/005679828 A, "Betulinic acid and dihydrobetulinic acid derivatives and uses therefor").

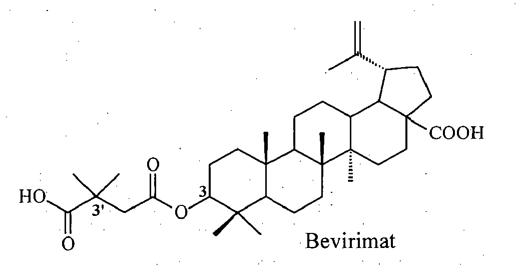

The results of further studies have shown that this was the first antiretroviral compound with a new mechanism of action, the so-called maturation inhibitor (T. Kanamoto et al., "Anti-human immunodeficiency virus activity of YK-FH312 (a betulinic acid derivative), a novel compound blocking viral maturation", Antimicrob. Agents Chemother., 2001, 45, 1225-1230; F. Li et al., "PA-457: A potent HIV inhibitor that disrupts core condensation by targeting a late step in Gag processing", Proc. Natl. Acad. Sci. USA, 2003, 100, 13555-13560). This compound, defined by Panacos Pharmaceuticals Inc. as "bevirimat" (also designated as PA-457, DSB, MPC-4326, YK-FH312), was subjected to clinical trials. In 2010 these studies were halted at phase IIb due to the emergence of resistance caused by the CA-SP1 protein mutation occurring in approximately 50% of HIV infected patients.

Recent studies on 2nd generation maturation inhibitors, which are not sensitive to the naturally occurring Gag protein polymorphism, confirm the high efficacy of the compound code-named BMS-955176 developed by Bristol-Myers Squibb Company (A. Regueiro-Ren et al., "Discovery of BMS-955176, a second generation HIV-1 maturation inhibitor with broad spectrum antiviral activity", ACS Med. Chem. Lett., 2016, 7, 568-572; US Pat. 2012/0142653 A1, "C-28 amides of modified C-3 betulinic acid derivatives as HIV maturation inhibitors"; US Pat. 2012/0142707 A1, "Modified C-3 betulinic acid derivatives as HIV maturation inhibitors"; US Pat. 2013/0029954 A1, "C-28 amines of C-3 modified betulinic acid derivatives as HIV maturation inhibitors"; US Pat. 2013/0035318 A1, "C-17 and C-3 modified triterpenoids with HIV maturation inhibitory activity").

A large number of betulin derivatives were synthesized and tested as a part of the research conducted by the GlaxoSmithKline Pharmaceuticals. Among them there was the compound code-named GSK 2828232, classified as a second generation HIV-1 maturation inhibitor (US Pat. 2013/0184263 A1, "Derivatives of betulin"; J.L Jeffrey et al., "GSK2838232, a second generation HIV-1 maturation inhibitor with an optimized virology profile". Conference on retroviruses and opportunistic infections, Seattle, Washington 2015, Abstract number 538; S. Xiao et al., "Recent progress in the antiviral activity and mechanism study of pentacyclic triterpenoids and their derivatives", Med. Res. Rev., 2018, 1-26).

In the search for new anti-HIV active substances that would demonstrate higher activity and selectivity and reduced toxicity, numerous modifications to the betulin structure at C3, C28, as well as C30 were carried out. Antiviral activity against HIV was determined in the case of several groups of betulin and betulinic acid derivatives.

Numerous 3-*O*-carboxyacyl, 28-*O*-carboxyacyl and 3,28-dicarboxyacylic derivatives were obtained in reactions of betulin and dihydrobetulin with dicarboxylic acids or their anhydrides (US Pat. 2001/6172110 B1, "Acylated betulin and dihydrobetulin derivatives, preparation thereof and use thereof"; US Pat. 2004/0131629 A1, "Monoacylated betulin and dihydrobetulin derivatives, preparation thereof and use thereof"). The most active were 3,28-disubstituted derivatives such as 3,28-di-*O*-(3',3'-dimethylglutaryl)betulin (EC₅₀ = 0.00066 µM, TI = 21,515) (I-C Sun et al., "Anti-AIDS agents. 32. Synthesis and anti-HIV activity of betulin derivatives", Bioorg. Med. Chem. Lett., 1998, 8, 1267-1272) and 3-*O*-(3',3'-dimethylsuccinyl)-28-*O*-(2",2"-dimethylsuccinyl)betulin (EC50 = 0.00087 µM, TI = 42,400) (Y. Kashiwada et al., "3,28-Di-O-(dimethylsuccinyl)betulin isomers as anti-HIV agents", Bioorg. Med. Chem. Lett., 2001, 11, 183-185)

Betulin conjugated with AZT via a triazol system did not show anti-HIV activity (I.D. Bori et al, "Anti-AIDS agents 88. Anti-HIV conjugates of betulin and betulinic acid with AZT prepared via click chemistry", Tetrahedron Lett., 2012, 53, 1987-1989), while conjugating 3-*O*-(3',3'-dimethylsuccinyl)betulin with AZT via an ester bond linker at C28 led to derivatives that demonstrated activity comparable to that of bevirimat (EC₅₀ within 0.040 - 0.098 µM,) (J. Xiong et al., "Conjugates of betulin derivatives with AZT as potent anti-HIV agents", Bioorg. Med. Chem., 2010, 18, 6451-6469).

In 2014, synthesis of new macrocyclic betulin derivatives was described in which the C30-C28 positions were linked by a chain containing an amide bond. However, these compounds showed poor activity against the HIV virus. Much more satisfying results were obtained in the case of derivatives of the 3-*O*-(3',3'-dimethylsuccinyl)betulinic acid (J. Tang et al., "Synthesis and biological evaluation of macrocyclized betulin derivatives as a novel class of anti-HIV-1 maturation inhibitors", Open Med. Chem. J., 2014, 8, 23-27). The synthesized derivatives represented a new class of HIV maturation inhibitors, like the 3-*O*-(3',3'-dimethylsuccinyl)betulin aldehyde and nitrile (C.R. Dorr et al., "Triterpene derivatives that inhibit human immunodeficiency virus type 1 replication", Bioorg. Med. Chem. Lett., 2011, 21, 542-545).

It was demonstrated through the studies on various betulinic acid derivatives that the dimethylsuccinyl moiety at C3 is crucial for inhibiting HIV maturation, while the presence of the side chain linked to the carboxylic group at C17 determines the compound's activity as an inhibitor of virus entry into the cell and membrane fusion. (X-T Liang, W-S Fang, "Triterpene betulinic acid derivatives as anti-HIV agents" in "Medicinal chemistry of bioactive natural products", New Jersey: Wiley-Interscience, 2006, 382, 379-397; K.H. Lee, "Discovery and development of natural product-derived chemotherapeutic agents based on a medicinal chemistry approach", J. Nat. Prod., 2010, 73, 500-516).

Betulinic acid derivatives modified at the C3-position by converting the hydroxyl group into a carbonyl, methoxy, amino, oxime group or by shifting its position from *β* to *α* or by its complete elimination leads to a reduction or loss of activity against HIV-1 (I-C Sun et al., "Anti-AIDS agents. 32. Synthesis and anti-HIV activity of betulin derivatives", Bioorg. Med. Chem. Lett., 1998, 8, 1267-1272; I-C Sun et al., "Anti-AIDS agents. 34. Synthesis and structure-activity relationships of betulin derivatives as anti-HIV agents", J. Med. Chem., 1998, 41, 4648-4657; M. Evers et al., "Betulinic acid derivatives: a new class of human immunodeficiency virus type 1 specific inhibitors with a new mode of action", J. Med. Chem., 1996, 39, 1056-1068; V.V. Grishko et al., "Functionalization, cyclization and antiviral activity of A-secotriterpenoids", Eur. J. Med. Chem., 2014, 83, 601-608; F. Soler et al., "Betulinic acid derivatives: a new class of specific inhibitors of human immunodeficiency virus type 1 entry", J. Med. Chem. 1996, 39, 1069-1083).

The structure of the 3-*O*-(3',3'-dimethylsuccinyl)betulinic acid, a compound that exhibits high activity against HIV-1, became a starting point for developing new derivatives. According to literature data, compounds with various acyl groups at C3 position have been described (F. Hashimoto et al., "Anti-AIDS agents-XXVII. Synthesis and anti-HIV activity of betulinic acid and dihydrobetulinic acid derivatives", Bioorg. Med. Chem., 1997, 5, 2133-2143; K. Qian et al., "Anti-AIDS agents 81. Design, synthesis, and structure-activity relationship study of betulinic acid and moronic acid derivatives as potent HIV maturation inhibitors", J. Med. Chem., 2010, 53, 3133-3141), 28-amide derivatives of bevirimat with carboxyl and/or amine terminal group (P. Coric et al., "Synthesis and biological evaluation of a new derivative of bevirimat that targets the Gag CA-SP1 cleavage site", Eur. J. Med. Chem., 2013, 62, 453-465; D. Gerrish et al., "Triterpene based compounds with potent anti-maturation activity against HIV-1", Bioorg. Med. Chem. Lett., 2008, 18, 6377-6380) as well as triazole-linked AZT conjugates (I.D. Bori et al. "Anti-AIDS agents 88. Anti-HIV conjugates of betulin and betulinic acid with AZT prepared via click chemistry" Tetrahedron Lett., 2012, 53, 1987-1989; A.T. Tuyet Dang et al., "New hybrids between triterpenoid acids and nucleoside HIV-RT inhibitors" Mendeleev Commun., 2015, 25, 96-98; T.A. Dang Thi et al., "Synthesis and cytotoxic evaluation of novel amide-triazole-linked triterpenoid-AZT conjugates" Tetrahedron Lett., 2015, 56, 218-224) or bevirimat analogs with amine substituents at C28 (E. Urano et al., "Alkyl amine bevirimat derivatives are potent and broadly active HIV-1 maturation inhibitors", Antimicrob. Agents Chemother., 2016, 60, 190-197).

In the following patent applications US Pat. 2011/0313191 A1 ("Preparation of pharmaceutical salts of 3-*O*-(3',3'-dimethylsuccinyl)betulinic acid"), US Pat. 2011/0224182 A1 ("Salts of (3-*O*-(3',3'-dimethylsuccinyl)betulinic acid and solid state forms thereof") and US Pat. 2011/0218204 A1 ("Pharmaceutically acceptable salts of novel betulinic acid derivatives") the salts of betulinic acid derivatives as well as various pharmaceutical compositions containing these active substances have been described.

Increased solubility of betulinic acid and improved activity against HIV were obtained by synthesizing ionic HIV protease inhibitors (H. Zhao et al., "Ionic derivatives of betulinic acid as novel HIV-1 protease inhibitors", J. Enzyme Inhib. Med. Chem., 2012, 27, 715-721).

Another area of research on new derivatives was the combination of two different mechanisms of action, achieved by synthesizing bifunctional compounds code-named LH 15 and LH 55, which contained a carboxyacyl group at the C3 position (required to act as a maturation inhibitor), while at the C28 position they contained carboxamide groups (acting as entry inhibitors) (C. Aiken and C.H. Chen, "Betulinic acid derivatives as HIV-1 antivirals", Trends Mol. Med., 2005, 11, 31-36; L. Huang et al., "Synthesis and anti-HIV activity of bi-functional betulonic acid derivatives", Bioorg. Med. Chem., 2006, 14, 2279-2289).

In the search for new derivatives active against HIV, compounds modified at C30 were also synthesized by introducing ester (I-C Sun et al., "Anti-AIDS agents. 34. Synthesis and structure-activity relationships of betulin derivatives as anti-HIV agents", J. Med. Chem., 1998, 41, 4648-4657), amine and ether groups (K. Qian et al., "Anti-AIDS agents. 78. Design, synthesis, metabolic stability assessment, and antiviral evaluation of novel betulinic acid derivatives as potent anti-human immunodeficiency virus (HIV) agents", J. Med. Chem., 2009, 52, 3248-3258) or by oxidizing to a formyl or epoxide group (F. Gutierrez-Nicolas et al., "Synthesis and anti-HIV activity of lupane and olean-18-ene derivatives. Absolute configuration of 19,20-epoxylupanes by VCD", J. Nat. Prod., 2011, 667-676).

The above literature review indicates that many different pharmacophoric groups have been introduced into the betulin molecule to obtain new substances with anti-HIV activity. However, compounds in which a phosphonate group is directly linked to the triterpene system have not been described so far. Antivirals currently in use include some phosphonic acid derivatives. Phosphonates have a P-C bond which, in comparison to the P-O-C bond, is stronger, which ensures a stronger and longer-acting effect of a given drug. Important antivirals include cidofovir (active against HSV-1, HSV-2, Epstein-Barr), adefovir and pradefovir (active against HBV), tenofovir (active against HIV-1, HIV-2, HBV), foscarnet (active against herpes, HIV), (K.K. Biron, "Antiviral drugs for cytomegalovirus diseases", Antiviral Research, 2006, 71, 154-163; M. Dracinsky et al., "Study of chemical stability of antivirally active 5-azacytosine acyclic nucleoside phosphonates using NMR spectroscopy", Bioorg. Med. Chem., 2008, 16, 6778-6782, P. Broganelli et al., "Intralesional cidofovir for the treatment of multiple and recalcitrant cutaneous viral warts", Dermatol. Ther., 2012, 25, 468-471). The US Pat. 2013/0225532 A1 ("Zanamivir phosphonate congeners with anti-influenza activity and determining oseltamivir susceptibility of influenza viruses") discloses phosphonic compounds active against wild strains of human and bird influenza H1N1, H5N1 and H3N2 viruses resistant to oseltamivir.

Phosphonates are compounds commonly found in living organisms. They constitute a group of compounds with antibiotic activity. An important example of such an antibiotic with broad application is fosfomycin which is isolated from various *Streptomyces* strains. It has strong antibacterial activity against both Gram-positive and Gram-negative pathogens (N. Roussos et al., "Clinical significance of the pharmacokinetic and pharmacodynamic characteristics of fosfomycin for the treatment of patients with systemic infections", Int. J. Antimicrob. Agents, 2009, 34, 506-515). Among phosphonic acid derivative antibiotics the fosmidomycin with antimalarial activity also deserves attention (B. Lell et al., "Fosmidomycin, a novel chemotherapeutic agent for malaria", Antimicrob. Agents Chemother., 2003, 47, 735-738). Rhizocticins, antifungal active antibiotics, belonging to the group of phosphonate antibiotics with a dipeptide structure, have been isolated from *Bacillus subtilis* cultures. Plumbemycins, tripeptide phosphonate antibiotics produced by *Streptomyces plumbeus,* exhibit antibacterial activity (B. Laber et al., "Inactivation of Escherichia coli threonine synthase by DL-Z-2-amino-5-phosphono-3-pentenoic acid", Arch. Microbiol., 1994, 161, 400-403).

Antibacterial activity is demonstrated by new phosphonates of chromene (M. Rajasekhar et al., "Green synthesis and bioactivity of 2-amino-4H-chromen-4-ylphosphonates", Chem. Pharm. Bull., 2012, 60, 854-858), as well as by diphenyl esters of *α*-hydroxy alkyl- and aryl- phosphonates (A.M.F. Phillips et al., "Synthesis and biological evaluation of α-hydroxyalkylphosphonates as new antimicrobial agents", Bioorg. Med. Chem. Lett., 2014, 24, 49-53).

For many years, bisphosphonate derivatives have been used in the treatment of osteoporosis. These include the following compounds: alendronate, etidronate, ibandronate, clodronate, pamidronate, risedronate, tiludronate and zoledronate (H. Fleisch, "Bisphosphonates: mechanisms of action", Endocr. Rev., 19, 1998, 80-100).

An important group of phosphonates are hydroxyphosphonates, among which human renin inhibitors with arterial hypotensive effect have been described (J.F. Dellaria et al., "New inhibitors of renin that contain novel phosphostatine Leu-Val replacements", J. Med. Chem., 1990, 33, 534-542; D.V. Patel et al., "a-Hydroxyphosphinyl-based inhibitors of human renin", J. Med. Chem., 1995, 38, 4557-4569). In recent years, studies have also been carried out on the antitumour activity of α-aminophosphonates, as well as phosphonates, which are already used as medications, e.g. foscarnet and bisphosphonates (K.A. Mungara et al., "Synthesis and antiproliferative activity of novel α-aminophosphonates", Chem. Pharm. Bull., 2012, 60, 1531-1537; C.B. Reddy et al., "PEG-SO3H catalyzed synthesis and cytotoxicity of α-aminophosphonates", Eur. J. Med. Chem., 2012, 47, 553-559; K. Rose, "Foscarnet reduces FGF2-induced proliferation of human umbilical vein endothelial cells and has antineoplastic activity against human anaplastic thyroid carcinoma cells", Biomed. Pharmacother., 2013, 67, 53-57; E.D. Carlo et al., "Mechanisms of the antitumor activity of human Vγ9Vδ2 T cells in combination with zoledronic acid in a preclinical model of neuroblastoma", Mol. Ther., 2013, 21, 1034-1043).

In the patent application of Gilead Sciences Inc. (US Pat. 2015/0025039 A1: "Antiviral phosphonate analogs"), which relates to phosphonate derivatives with antiviral activity, numerous basic structures are disclosed, including betulinic acid and dihydrobetulinic acid. However, the phosphonate group is not linked directly to the triterpene system but is located in the long substituent chain at C3 or C28 positions. US Pat. 2016/9428542 B2 ("Lupane triterpenoid derivatives and pharmaceutical use thereof') describes the anti-HIV active lupane derivatives, which are bevirimat analogs with the -phosphono-substituted aromatic groups linked by various linkers at the C17 position. Derivatives of betulin with phosphonate groups in a large substituent at the C3 and C28 position are also disclosed in US Pat. 2012/0046291 A1 ("Extended Triterpene Derivatives").

For the first time acetylenic derivatives of betulin 29-phosphonate with antitumour activity were described in the Polish patent PL227790 ("*Phosphonates of acetylenic betulin derivatives with antitumour activity, method for their production and their application*") and in the patent application EP2016/16183580 ("*Phosphonates of acetylenic betulin derivatives with anticancer activity, method for their production and their application*") and in the publication by E. Chrobak et al., ("Betulin phosphonates; synthesis, structure, and cytotoxic activity", Molecules, 2016, 21, 1-13). The introduction of a phosphonate group, being a bioisosteric replacement for a phosphoric group, ensures higher stability of phosphonates under enzymatic hydrolysis conditions. Free phosphonic acids, due to the high negative charge of the phosphoryl moiety, penetrate less effectively through biological membranes as compared to the corresponding phosphonate esters. Therefore, the synthesis of phosphonate esters is becoming increasingly important in the design of new phosphonates.

The main goal of the inventors was to develop new compounds with antiviral activity, the structure of which is based on the structure of the 3-*O*-(3',3'-dimethylsuccinyl)betulinic acid molecule modified in the isopropenyl group by introducing a phosphonate substituent. The objective, as well as an advantage of the presented method is also the use of commercially available reagents, running the process under mild conditions and, above all, using betulin as the main substrate, which can be easily obtained, e.g. from birch bark, which is waste in the production of paper.

The essence of the invention are the new phosphonate derivatives of the 3-carboxyacylbetulinic acid with antiviral activity, of formula **1A** or of formula **1B,** wherein:
R is an alkyl group (C₁-C₄),
R₁ is an alkyl group (C₁-C₄),
R₂ is a hydroxyl or carbonyl or acetyloxy or carboxyacyloxy group,
R₃ is an acetyloxymethyl or hydroxymethyl or carboxyl group.

In the method for the preparation of compounds represented by general formula **1A** or **1B** according to the invention, the starting material for the synthesis of phosphonate derivatives of 3-acetylbetulinic acid is the compound of formula **2A** obtained by multi-stage synthesis from betulin (most preferably derived from birch bark) and a compound of formula **2B,** a vinyl isomer, formed by the hydrolysis of compound **2A** (compounds **2A** and **2B** are described in patent PL 227790, 2017, "Phosphonates of acetylenic betulin derivatives with antitumour activity, method for their production and their application").

The essence of the invention is also the method for preparation of the 30-phosphono derivatives of the 3-carboxyacylbetulinic acid with antiviral activity, of formula **1A,** wherein:
R is an alkyl group (C₁-C₄),
R₁ is an alkyl group (C₁-C₄),
R₂ is a hydroxyl or carbonyl or acetyloxy or carboxyacyloxy group,
R₃ is an acetyloxymethyl or hydroxymethyl or carboxyl group,
characterized in that it proceeds in the following steps:
**(a)** compound of formula **2A,** wherein:
   R is an alkyl group (C₁-C₄),
   R₁ is an alkyl group (C₁-C₄),
   is subjected to alkaline hydrolysis in the presence of an aqueous solution of a metal hydroxide selected from KOH and NaOH, with a concentration of 2-4 N, in an organic solvent or in a mixture of organic solvents, wherein the reaction is carried out at room temperature for 0.5 to 4 hours, resulting in 3-acetylbetulin 30-phosphonate,
**(b)** the product of step (a) is reacted with the Jones reagent taken in an amount of 1 to 4 ml per 1 mmol of the product of step (a), in at least 10 ml of an organic solvent per 1 mmol of the product of step (a), resulting in 30-phosphonate of 3-acetylbetulinic acid,
**(c)** the product of step (b) is subjected to alkaline hydrolysis in the presence of an aqueous solution of a metal hydroxide selected from KOH and NaOH, with a concentration of 2-4 N, or an aqueous solution of potassium carbonate K₂CO₃ with a concentration of 1-2 M, in an organic solvent, wherein the reaction is carried out at room temperature for 3 to 24 hours, resulting in 30-phosphonate of betulinic acid,
**(d)** the product of step (c) is reacted with a dicarboxylic acid anhydride or with a dicarboxylic acid, at a molar ratio of between 1:1 and 1:10, for at least 30 minutes.

Preferably, the reaction of step (a) is carried out in a solvent selected from methanol and ethanol, or in a mixture of tetrahydrofuran and methanol (1:3 to 3:1, v/v), or in a mixture of methanol and chloroform (preferably 1:1, v/v).

Preferably, step (b), i.e. the reaction of the product of step (a) with the Jones reagent, is carried out in an acidic medium at a temperature of from -10°C to room temperature, most preferably at a temperature of 20 to 24°C.

Preferably, the organic solvent used in step (b) is acetone or dichloromethane or a mixture thereof, most preferably at the solvents ratio of 1:1, v/v.

Preferably, the reaction of step (c) is carried out in a solvent selected from methanol and ethanol, or in a mixture of tetrahydrofuran and methanol (1:3 to 3:1, v/v).

Preferably, step (d), i.e. the reaction of the product of step (c) with a dicarboxylic acid anhydride or with a dicarboxylic acid, is carried out in an organic solvent in an amount necessary to dissolve the product of step (c) in the presence of 4-dimethylaminopyridine at a molar ratio of 1:1 to 1:2 based on the product of step (c), preferably in a microwave reactor, preferably at a temperature of 120 to 160° C, whereas the organic solvent used is one selected from the group consisting of: pyridine, ethylene chloride, chloroform, toluene, diethyl ether, dimethylsulfoxide, acetonitrile, tetrahydrofuran and dioxane.

The essence of the invention is also a method for the preparation of 29-phosphonic derivatives of 3-carboxyacylbetulinic acid with antiviral activity, of formula **1B,** wherein:
R is an alkyl group (C₁-C₄),
R₁ is an alkyl group (C₁-C₄),
R₂ is a hydroxyl or carbonyl or acetyloxy or carboxyacyloxy group,
R₃ is an acetyloxymethyl or hydroxymethyl or carboxyl group,
characterized in that it proceeds in the following steps:
**(a)** compound of formula **2B,** wherein:
   R is an alkyl group (C₁-C₄),
   R₁ is an alkyl group (C₁-C₄),
   is reacted with the Jones reagent taken in an amount of 2 to 6 ml per 1 mmol of the compound of formula **2B,** in at least 10 ml of an organic solvent per 1 mmol of the compound of formula **2B,** resulting in 29-phosphonate of betulonic acid,
**(b)** the product of step (a) is reduced by sodium or lithium borohydride, resulting in 29-phosphonate of betulinic acid,
**(c)** the product of step (b) is reacted with a dicarboxylic acid anhydride or with a dicarboxylic acid, at a molar ratio of between 1:2.5 to 1:10, for at least 30 minutes.

Preferably, step (a), i.e. the reaction of the product of formula **2B** with the Jones reagent, is carried out in an acidic medium at a temperature of from -10°C to room temperature, most preferably at a temperature of 20 to 24°C.

Preferably, the organic solvent used in step (a) is acetone or dichloromethane or a mixture thereof, most preferably at the solvents ratio of 1:1, v/v.

Preferably, step (b), i.e. the reaction of the product of step (a) with sodium or lithium borohydride at a molar ratio of at least 1:1.3 is carried out in an organic solvent at a ratio of at least 10 ml of the solvent per 1 mmol of the product of step (a), at room temperature, for at least 30 minutes, whereas the organic solvent used is one selected from the group consisting of: tetrahydrofuran, diethyl ether, isopropanol, dioxane, ethanol and methanol.

Preferably, step (c), i.e. the reaction of the product of step (b) with a dicarboxylic acid anhydride or with a dicarboxylic acid, is carried out in an organic solvent in an amount necessary to dissolve the product of step (b) in the presence of 4-dimethylaminopyridine at a molar ratio of 1:1 to 1:2 based on the product of step (b), preferably in a microwave reactor, preferably at a temperature of 120 to 160°C, whereas the organic solvent used is one selected from the group consisting of: pyridine, methylene chloride, chloroform, toluene, diethyl ether, dimethylsulfoxide, acetonitrile, tetrahydrofuran and dioxane.

The essence of the invention is also the use of phosphonate derivatives of 3-carboxyacylbetulinic acid with antiviral activity, of formula **1A** or **1B,** wherein:
R is an alkyl group (C₁-C₄),
R₁ is an alkyl group (C₁-C₄),
R₂ is a hydroxyl or carbonyl or acetyloxy or carboxyacyloxy group,
R₃ is an acetyloxymethyl or hydroxymethyl or carboxyl group,
in the manufacture of pharmaceutical agents for suppressing HIV-1 replication.

The method of preparing phosphonate derivatives of 3-carboxyacylbetulinic acid according to the invention uses readily available starting materials. The structure of the compounds was confirmed by ¹H NMR, ¹³C NMR, ³¹P NMR and IR spectroscopy.

The compounds according to the invention exhibit activity against HIV (Human Immunodeficiency Virus), with the values of CC₅₀, IC₅₀ and TI close to or better than those of the prior art 3-(3',3'-dimethylsuccinyl)betulinic acid.

The method of the invention is described more in detail in the examples below, which do not limit the scope of its protection in any way.

### Example 1.

Preparation of 3-acetylobetulin 30-phosphonate of formula **1A** (R = R₁ = Me or Et, R₂ = AcO, R₃ = CH₂OH).

Compound of formula **2A,** where R = R₁ = Me or Et (0.66 g; 1 mmol), is added to 50 ml of a 0.2 M solution of sodium hydroxide in a mixture of methanol, tetrahydrofuran and water (1:2:1, v/v), and stirred at room temperature for 0.5 hour. When the reaction is completed, 30 ml of methylene chloride is added to the mixture, which is afterwards washed with a 10% solution of hydrochloric acid and with water. The organic layer is dried with anhydrous sodium sulphate. Upon purifying the crude product by column chromatography (SiO₂, chloroform/ethanol, 15:1, v/v) the product of formula **1A** is obtained, wherein R = R₁ = Me or Et, R₂ = AcO, R₃ = CH₂OH.
**a)** 3-*O*-Acetyl-30-dimethoxyphosphorylbetulin **1A** (R = R₁ = Me, R₂ = AcO, R₃ = CH₂OH)
   Yield: 68%. M.p. 113-114°C.
   TLC (methylene chloride/ethanol, 15:1, v/v): R_{f} = 0.24.
   Spectroscopic data of the product obtained:
      ¹H NMR (CDCl₃) δ (ppm): 0.80 (m, 1H, H5); 0.85 (s, 3H, CH₃); 0.86 (s, 6H, 2 x CH₃); 0.99 (s, 3H, CH₃); 1.04 (s, 3H, CH₃); 0.95 - 2.20 (m, 25H, CH, CH₂); 2.06 (s, 3H, C(O)CH₃); 2.4 (m, 1H, H19); 2.58 (m, 2H, H30); 3.33 (m, 1H, H28); 3.78 (s, 3H, OCH₃); 3.80 (s, 3H, OCH₃); 3.81 (m, 1H, H28); 4.49 (m, 1H, H3); 5.03 (m, 1H, H29); 5.05 (m, 1H, H29);
      ¹³C NMR (CDCl₃) δ (ppm): 14.7; 16.0; 16.2; 16.5; 18.2; 20.9; 21.3; 23.7; 26.5; 27.0; 27.9; 29.5; 33.6; 33.9; 34.2; 37.0; 37.1; 37.6; 37.8; 38.4; 40.9; 41.0; 42.7; 47.8; 50.0; 50.3; 52.7; 55.4; 60.5; 80.9; 112.9; 144.6; 151.1; 171.0.
      ³¹P NMR (CDCl₃) δ (ppm): 30.3.
      IR (KBr) v (cm⁻¹): 3445 (O-H), 1732 (C=O), 1246 (P=O), 1031 (P-O-C), 806 (P-C).
**b)** 3-*O*-Acetyl-30-diethoxyphosphorylbetulin **1A** (R = R₁ = Et, R₂ = AcO, R₃ = CH₂OH) Yield: 61%. M.p. 118-122°C.
   TLC (chloroform/ethanol, 15:1, v/v): R_{f} = 0.37.
   Spectroscopic data of the product obtained:
      ¹H NMR (CDCl₃) δ (ppm): 0.72 (m, 1H, H5); 0.76 (s, 3H, CH₃); 0.78 (s, 6H, 2 x CH₃); 0.90 (s, 3H, CH₃); 0.95 (s, 3H, CH₃); 1.2 - 2.05 (m, 24 H, CH, CH₂); 1.25 (m, 6H, 2 x OCH₂CH₃); 1.98 (s, 3H, C(O)CH₃); 2.4 (m, 1H, H19); 2.5 (m, 2H, H30); 3.25 (m, 1H, H28); 3.65 (m, 1H, H28); 4.05 (m, 4H, 2 x OCH₂CH₃); 4.4 (m, 1H, H3); 4.92 (m, 1H, H29); 4.96 (m, 1H, H29). ¹³C NMR (CDCl₃) δ (ppm): 14.7; 16.0; 16.2; 16.4; 18.1; 18.4; 20.9; 21.3; 23.7; 26.9; 27.0; 27.9; 29.2; 33.6; 33.9; 34.1; 37.0; 37.1; 37.6; 37.8; 38.4; 40.9; 41.0; 42.7; 47.7; 50.1; 50.2; 55.3; 58.5; 60.5; 61.9; 80.9; 112.9; 151.1; 171.1.
      ³¹P NMR (CDCl₃) δ (ppm): 27.8
      IR (KBr) v (cm⁻¹): 3439 (O-H), 1732 (C=O), 1246 (P=O), 1028 (P-O-C), 648 (P-C).

### Example 2.

Preparation of phosphonate derivatives of 3-acetylbetulinic acid of formula **1A** (R = R₁ = Me or Et, R₂ = AcO, R₃ = COOH) or of betulonic acid of formula **1B** (R = R₁ = Me or Et, R₂ = O, R₃ = COOH).

Cold solution of Jones reagent (4 ml) is gradually added dropwise to a cooled solution of 1 mmol of compound **1A** (R = R₁ = Me or Et, R₂ = AcO, R₃ = CH₂OH) or **2B** (R = R₁ = Me or Et) in 14 ml of acetone at a rate that prevents temperature rise above 0°C. Stirring is continued for 1.5 hour at room temperature. The flask is then placed in a water bath at 10°C, 3 ml of ethanol is gradually added and stirring is continued for 30 minutes. After that the mixture is poured into 15 ml of water mixed with crushed ice. The suspension formed is extracted with methylene chloride. The extracts are dried with anhydrous sodium sulphate and, after concentrating, are purified by column chromatography (SiO₂, methylene chloride/ethanol, 15:1 v/v) to obtain compounds **1A** (R = R₁ = Me or Et, R₂ = AcO, R₃ = COOH) or **1B** (R = R₁ = Me or Et, R₂ = O, R₃ = COOH).
**a)** 3-*O*-Acetyl-30-dimethoxyphosphorylbetulinic acid **1A** (R = R₁ = Me, R₂ = AcO, R₃ = COOH)
   Yield: 39%. M.p. 173-174°C.
   TLC (chloroform/ethanol, 15:1, v/v): R_{f} = 0.32.
   Spectroscopic data of the product obtained:
      ¹H NMR (CDCl₃) δ (ppm): 0.81 (m, 1H, H5); 0.84 (s, 3H, CH₃); 0.86 (s, 6H, 2 x CH₃); 0.94 (s, 3H, CH₃); 0.98 (s, 3H, CH₃); 0.95 - 2.35 (m, 24 H, CH, CH₂); 2.06 (s, 3H, C(O)CH₃); 2.61 (m, 2H, H30); 3.02 (m, 1H, H19); 3.77 (s, 3H, OCH₃); 3.79 (s, 3H, OCH₃); 4.49 (m, 1H, H3); 5.06 (m, 1H, H29); 5.08 (m, 1H, H29);
      ¹³C NMR (CDCl₃) δ (ppm): 14.7; 16.0; 16.2; 16.5; 18.2; 21.0; 21.4; 23.7; 26.5; 27.9; 29.7; 32.1; 34.3; 36.7; 37.1; 37.8; 38.1; 38.3; 38.4; 40.7; 42.4; 46.21; 50.4; 50.6; 52.7; 52.8; 55.4; 56.3; 58.5; 80.9; 113.0; 144.7; 171.1; 180.6.
      ³¹P NMR (CDCl₃) δ (ppm): 30.6.
      IR (KBr) v (cm⁻¹): 3005 (O-H), 1732 (C=O), 1248 (P=O), 1031 (P-O-C), 731 (P-C).
**b)** 3-*O*-Acetyl-30-diethoxyphosphorylbetulinic acid **1A** (R = R₁ = Et, R₂ = AcO, R₃ = COOH)
   Yield: 63%. M.p. 231-233°C.
   TLC (chloroform/ethanol, 15:1, v/v): R_{f} = 0.35.
   Spectroscopic data of the product obtained:
      ¹H NMR (CDCl₃) δ (ppm): 0.80 (m, 1H, H5); 0.84 (s, 3H, CH₃); 0.86 (s, 6H, 2 x CH₃); 0.94 (s, 3H, CH₃); 0.99 (s, 3H, CH₃); 1.2 - 2.35 (m, 24 H, CH, CH₂); 1.25 (m, 6H, 2 x OCH₂CH₃); 2.06 (s, 3H, C(O)CH₃); 2.6 (m, 2H, H30); 3.02 (m, 1H, H19); 4.14 (m, 4H, 2 x OCH₂CH₃); 4.49 (m, 1H, H3); 5.04 (m, 1H, H29); 5.08 (m, 1H, H29);
      ¹³C NMR (CDCl₃) δ (ppm): 14.7; 16.0; 16.2; 16.4; 18.2; 18.4; 21.0; 21.3; 23.7; 27.9; 29.6; 30.9; 32.1; 34.3; 36.3; 36.7; 37.1; 37.8; 38.1; 38.3; 38.4; 40.7; 42.4; 50.3; 50.4; 55.4; 56.1; 56.2; 58.5; 61.9; 80.9; 112.8; 151.1; 171.0; 178.5.
      ³¹P NMR (CDCl₃) δ (ppm): 28.0.
      IR (KBr) v (cm⁻¹): 3420 (O-H), 1738 (C=O), 1246 (P=O), 1024 (P-O-C), 753 (P-C).
**c)** 29-Dimethoxyphosphorylbetulonic acid **1B** (R = R₁ = Me, R₂ = O, R₃ = COOH) Yield: 31%. M.p. 230-231°C.
   TLC (chloroform/ethanol, 15:1, v/v): R_{f} = 0.21.
   Spectroscopic data of the product obtained:
      ¹H NMR (CDCl₃) δ (ppm): 0.94 (s, 3H, CH₃); 0.98 (s, 3H, CH₃); 1.00 (s, 3H, CH₃); 1.01 (s, 3H, CH₃); 1.06 (s, 3H, CH₃); 0.93 - 2.36 (m, 23 H, CH, CH₂); 2.08 (broadened s, 3H, H-30); 2.44 (m, 1H, H-2); 2.51 (m, 1H, H-2); 3.14 (m, 1H, H-19); 3.71 (s, 3H, OCH₃); 3.72 (s, 3H, OCH₃); 5.48 (d, 1H, ²*J*_{PH}=18.0 Hz, H-29).
      ¹³C NMR (CDCl₃) δ (ppm): 14.6; 15.8; 16.0; 19.6; 21.0; 21.4; 25.9; 26.7; 29.6; 30.6; 31.9; 33.5; 34.1; 36.7; 37.0; 38.2; 39.6; 40.6; 42.5; 47.3; 49.7; 50.0; 50.4; 54.8; 56.3; 167.3; 179.9; 218.2.
      ³¹P NMR (CDCl₃) δ (ppm): 21.6.
      IR (KBr, cm⁻¹) v: 3496 (O-H), 1705 (C=O), 1178 (P=O), 1059 (P-O-C), 735 (P-C).
**d)** 30-Dimethoxyphosphorylbetulonic acid **1A** (R = R₁ = Me, R₂ = O, R₃ = COOH) Yield: 28%. M.p. 244-247°C.
   TLC (methylene chloride/ethanol, 15:1, v/v): R_{f} = 0.23.
   Spectroscopic data of the product obtained:
      ¹H NMR (CDCl₃) δ (ppm): 0.94 (s, 3H, CH₃); 0.98 (s, 3H, CH₃); 1.00 (s, 3H, CH₃); 1.01 (s, 3H, CH₃); 1.06 (s, 3H, CH₃); 0.93 - 2.55 (m, 25H, CH, CH₂); 2.63 (m, 2H, H-30); 3.01 (m, 1H, H-19); 3.78 (s, 3H, OCH₃); 3.79 (s, 3H, OCH₃); 5.06 (br. s, 1H, H-29); 5.08 (br. s, 1H, H-29).
      ¹³C NMR (CDCl₃) δ (ppm): 14.7; 15.8; 16.0; 19.6; 21.0; 21.5; 25.9; 26.5; 26.7; 29.7; 30.6; 32.0; 33.6; 34.1; 36.7; 37.0; 38.4; 39.6; 40.7; 42.5; 47.3; 49.8; 50.0; 50.4; 52.0; 54.9; 56.3; 167.3; 180.2; 218.2.
      ³¹P NMR (CDCl₃) δ (ppm): 30.5.
      IR (KBr, cm⁻¹) v: 3496 (O-H), 1705 (C=O), 1236 (P=O), 1032 (P-O-C), 735 (P-C).
**e)** 29-Diethoxyphosphorylbetulonic acid **1B** (R = R₁ = Et, R₂ = O, R₃ = COOH)
   Yield: 31%. M.p. 244-245°C.
   TLC (chloroform/ethanol, 15:1, v/v): R_{f} = 0.56.
   Spectroscopic data of the product obtained:
      ¹H NMR (CDCl₃) δ (ppm): 0.94 (s, 3H, CH₃); 0.99 (s, 3H, CH₃); 1.00 (s, 3H, CH₃); 1.04 (s, 3H, CH₃); 1.10 (s, 3H, CH₃); 1.33 (m, 6H, 2 x OCH₂CH₃); 0.93 - 2.36 (m, 22 H, CH, CH₂); 2.08 (d, *J*=3Hz, 3H, H-30); 2.45 (m, 1H, H-2); 2.50 (m, 1H, H-2); 3.13 (m, 1H, H-19); 4.06 (m, 4H, 2 x OCH₂CH₃); 5.50 (d, 1H, ²*J*_{PH}=18.6 Hz, H-29).
      ¹³C NMR (CDCl₃) δ (ppm): 14.6; 15.8; 16.0; 16.4; 19.6; 21.0; 21.3; 25.9; 26.7; 29.6; 30.5; 32.0; 33.6; 34.1; 36.9; 37.0; 38.2; 39.5; 40.6; 42.5; 47.3; 49.7; 50.0; 50.5; 50.7; 54.9; 56.2; 61.2; 61.3; 111.2; 167.5; 179.8; 218.0.
      ³¹P NMR (CDCl₃) δ (ppm): 18.75.
      IR (KBr, cm⁻¹) v: 1717 (O-H), 1701 (C=O), 1236 (P=O), 972 (P-O-C), 799 (P-C).

### Example 3.

Preparation of 29-dialkoxyphosphoryl derivatives of betulinic acid of formula **1B** (R = R₁ = Me or Et, R₂ = OH, R₃ = COOH).

Sodium tetrahydroborate (NaBH₄; 0.028 g, 0.72 mmol) is added to a suspension of 0.12 g (ca. 0.2 mmol) of the compound of formula **1B** (R = R₁ = Me or Et, R₂ = O, R₃ = COOH) in 8 ml of tetrahydrofuran and stirred at room temperature for 2 hours. The post-reaction mixture is diluted with 15 ml of water and is then extracted with methylene chloride. The extracts are dried with anhydrous sodium sulphate and concentrated till dry. The crude product is purified by column chromatography (SiO₂, methylene chloride/ethanol, 15:1 v/v) to obtain compound **1B** (R = R₁ = Me or Et, R₂ = OH, R₃ = COOH).
**a)** 29-Dimethoxyphosphorylbetulinic acid **1B** (R = R₁ = Me, R₂ = OH, R₃ = COOH).
   Yield: 62%. M.p. 234-237°C.
   TLC (methylene chloride/ethanol, 15:1, v/v): R_{f} = 0.23.
   Spectroscopic data of the product obtained:
      ¹H NMR (CDCl₃) δ (ppm): 0.70 (m, 1H, H-5); 0.77 (s, 3H, CH₃); 0.84 (s, 3H, CH₃); 0.94 (s, 3H, CH₃); 0.99 (s, 6H, 2 x CH3); 0.90 - 2.35 (m, 25H, CH, CH₂); 2.08 (d, *J*=3Hz, 3H, H-30); 3.13 (m, 1H, H-19); 3.22 (m, 1H, H-3); 3.70 (s, 3H, OCH₃); 3.72 (s, 3H, OCH₃); 5.47 (d, 1H, ²*J*_{PH}=19.2 Hz, H-29).
      ¹³C NMR (CDCl₃) δ (ppm): 14.0; 14.7; 15.4; 16.0; 16.1; 18.3; 20.8; 25.9; 27.3; 28.0; 29.6; 30.6; 31.0; 32.0; 34.3; 37.0; 37.2; 38.1; 38.7; 38.9; 40.7; 42.4; 50.1; 50.4; 51.9; 55.3; 56.2; 68.3; 78.9; 115.0; 170.2; 179.2.
      ³¹P NMR (CDCl₃) δ (ppm): 21.6.
      IR (KBr, cm⁻¹) v: 3467 (O-H), 1712 (C=O), 1217 (P=O), 1033 (P-O-C), 813 (P-C).
**b)** 29-Diethoxyphosphorylbetulinic acid **1B** (R = R₁ = Et, R₂ = OH, R₃ = COOH) Yield: 50%. M.p. 271-273°C.
   TLC (chloroform/ethanol, 15:1, v/v): R_{f} = 0.31.
   Spectroscopic data of the product obtained:
      ¹H NMR (CDCl₃) δ (ppm): 0.71 (m, 1H, H-5); 0.78 (s, 3H, CH₃); 0.84 (s, 3H, CH₃); 0.95 (s, 3H, CH₃); 0.99 (s, 6H, 2 x CH₃); 1.34 (m, 6H, 2 x OCH₂CH₃); 0.90-2.10 (m, 24H, CH, CH₂); 2.08 (d, *J*=3Hz, 3H, H-30); 3.12 (m, 1H, H-19); 3.22 (m, 1H, H-3); 4.06 (m, 4H, 2 x OCH₂CH₃); 5.49 (d, 1H, ²*J*_{PH}=18.6 Hz, H-29).
      ¹³C NMR (CDCl₃) δ (ppm): 14.7; 15.4; 16.0; 16.1; 16.4; 17.1; 18.3; 20.8; 26.0; 27.3; 28.0; 29.6; 30.5; 32.0; 34.3; 37.0; 37.2; 38.1; 38.1; 38.7; 38.9; 40.7; 42.4; 50.0; 50.4; 50.7; 55.3; 56.2; 61.2; 111.2; 167.7; 179.7.
      ³¹P NMR (CDCl₃) δ (ppm): 18.80.
      IR (KBr, cm⁻¹) v: 3417 (O-H), 1707 (C=O), 1227 (P=O), 1027 (P-O-C), 751 (P-C).

### Example 4.

Preparation of 30-dialkoxyphosphorylbetulinic acids of formula **1A** (R = R₁ = Me or Et, R₂ = OH, R₃ = COOH).

Compound of formula **1A,** where R = R₁ = Me or Et, R₂ = AcO, R₃ = COOH (1 mmol), is added to 60 ml of a 0.2 M solution of sodium hydroxide in a mixture of methanol, tetrahydrofuran and water (1:2:1, v/v), and stirred at room temperature for 24 hours. When the reaction is completed, the volatile constituents are evaporated at reduced pressure, and 30 ml of methylene chloride is added to the evaporation residue, which is afterwards washed with a 10% solution of hydrochloric acid and with water. The organic layer is dried with anhydrous sodium sulphate. Upon purifying the crude product by column chromatography (SiO₂, chloroform/ethanol, 15:1, v/v) the product of formula **1A** is obtained, wherein R = R₁ = Me or Et, R₂ = OH, R₃ = COOH.
**a)** 30-Dimethoxyphosphorylbetulinic acid **1A** (R = R₁ = Me or Et, R₂ = OH, R₃ = COOH).
   Yield: 52%. M.p. 223-225°C.
   TLC (methylene chloride/ethanol, 15:1, v/v): R_{f} = 0.19.
   Spectroscopic data of the product obtained:
      ¹H NMR (CDCl₃) δ (ppm): 0.7 (m, 1H, H5); 0.77 (s, 3H, CH₃); 0.84 (s, 3H, CH₃); 0.94 (s, 3H, CH₃); 0.98 (s, 3H, CH₃); 0.99 (s, 3H, CH₃); 1.2 - 2.3 (m, 25H, CH, CH₂); 2.61 (m, 2H, H30); 2.97 (m, 1H, H19); 3.20 (m, 1H, H3); 3.78 (s, 6H, 2 x OCH₃); 4.94 (m, 1H, H29); 4.99 (m, 1H, H29).
      ¹³C NMR (CDCl₃) δ (ppm): 14.7; 15.4; 16.0; 16.1; 18.3; 21.0; 27.4; 28.0; 29.7; 31.0; 32.1; 34.3; 36.7; 37.2; 38.3; 38.7; 38.9; 40.7; 42.4; 50.5; 51.9; 52.7; 52.8; 55.3; 56.2; 58.5; 79.0; 113.0; 151.3; 178.8.
      ³¹P NMR (CDCl₃) δ (ppm): 30.5.
      IR (KBr) v (cm⁻¹): 3466 (O-H), 1681 (C=O), 1242 (P=O), 1031 (P-O-C), 860 (P-C).
**b)** 30-Diethoxyphosphorylbetulinic acid **1A** (R = R₁ = Et, R₂ = OH, R₃ = COOH)
   Yield: 66%. M.p. 266-268°C.
   TLC (chloroform/ethanol, 15:1, v/v): R_{f} = 0.31.
   Spectroscopic data of the product obtained:
      ¹H NMR (CDCl₃) δ (ppm): 0.6 (m, 1H, H5); 0.68 (s, 3H, CH₃); 0.75 (s, 3H, CH₃); 0.85 (s, 3H, CH₃); 0.90 (s, 6H, 2 x CH₃); 1.2 - 2.3 (m, 25H, CH, CH₂); 1.25 (m, 6H, 2 x OCH₂CH₃); 2.5 (m, 2H, H30); 2.93 (m, 1H, H19); 3.11 (m, 1H, H3); 4.03 (m, 4H, 2 x OCH₂CH₃); 4.94 (m, 1H, H29); 4.99 (m, 1H, H29).
      ¹³C NMR (CDCl₃) δ (ppm): 14.7; 15.4; 16.1; 16.4; 18.3; 21.0; 27.4; 28.0; 29.7; 30.9; 32.1; 34.3; 36.7; 37.2; 38.3; 38.7; 38.9; 40.7; 42.4; 50.5; 55.3; 56.2; 61.8; 62.0; 79.0; 112.9; 178.8. ³¹P NMR (CDCl₃) δ (ppm): 27.9.
      IR (KBr) v (cm⁻¹): 3431 (O-H), 1681 (C=O), 1236 (P=O), 1024 (P-O-C), 751 (P-C).

### Example 5.

Preparation of 3-carboxyacyl derivatives of phosphonobetulinic acid of formula **1A** and **1B** [R = R₁ = Et, R₂ = HOOCC(CH₃)₂CH₂C(O)O, HOOCCH₂C(CH₃)₂CH₂C(O)O, HOOCC(CH₃)₂CH₂CH₂C(O)O, R₃ = COOH].

*N,N*-Dimethylaminopyridine (DMAP; 1.5 mmol, 190 mg) and 5 mmol of appropriate acid anhydride (2,2-dimethylsuccinyl anhydride or 3,3-dimethylglutaric anhydride or 2,2-dimethylglutaric anhydride) is added to a solution of 1 mmol of appropriate acid 1A or **1B** (R = R₁ = Et, R₂ = OH, R₃ = COOH) in 2 ml pyridine. The reaction vessel is placed in a microwave reactor and the reaction is carried out for 1.5 hour at a temperature of 130-160° C at maximum wave power (300W). After cooling, the mixture is diluted with 25 ml of ethyl acetate, then washed with a 20% hydrochloric acid solution and with water. The organic layer is dried with anhydrous sodium sulphate and concentrated till dry in a vacuum evaporator. The crude product is purified by column chromatography (SiO₂, methylene chloride/ethanol, 15:1 v/v) to obtain compound of formula **1A** or **1B** (R = R₁ = Et, R₂ = HOOCC(CH₃)₂CH₂C(O)O, HOOCCH₂C(CH₃)₂CH₂C(O)O, HOOCC(CH₃)₂CH₂CH₂C(O)O, R₃ = COOH]
**a)** 30-Diethoxyphosphoryl-3-*O*-(3,3'-dimethylsuccinyl)betulinic acid **1A** [R = R₁ = Et, R₂ = HOOCC(CH₃)₂CH₂C(O)O, R₃ = COOH]
   Yield: 20% M.p. 122-126°C.
   TLC (chloroform/ethanol, 15:1, v/v): R_{f} = 0.21.
   Spectroscopic data of the product obtained:
      ¹H NMR (CDCl₃) δ (ppm): 0.78 (m, 1H, H5); 0.81 (s, 3H, CH₃); 0.85 (s, 3H, CH₃); 0.90 (s, 3H, CH₃); 0.99 (s, 6H, CH₃); 1.28 (s, 3H, CH₃); 1.30 (s, 3H, CH₃); 1.32 (m, 6H, 2 x OCH₂CH₃); 0.8 - 2.4 (m, 26H, CH, CH₂); 2.48 (d, 1H, *J* = 15.6 Hz, CH); 2.64 (m, 2H, H30); 2.89 (d, 1H, *J* = 15.6 Hz, CH); 3.13 (m, 1H, H19); 4.13 (m, 4H, 2 x OCH₂CH₃); 4.55 (m, 1H, H3); 5.03 (m, 1H, H29); 5.07 (m, 1H, H29).
      ¹³C NMR (CDCl₃) δ (ppm): 14.1; 14.5; 16.4; 16.6; 16.9; 17.4; 18.4; 19.7; 21.0; 23.6; 24.2; 26.9; 27.3; 28.5; 29.8; 30.9; 32.0; 33.9; 36.9; 37.9; 38.3; 40.5; 40.7; 42.4; 49.6; 50.6; 56.7; 61.8; 61.9; 81.4; 110.6; 126.7; 170.6; 182.4; 183.1.
      ³¹P NMR (CDCl₃) δ (ppm): 27.9
      IR (CDCl₃) v (cm⁻¹): 1705 (C=O), 1219 (P=O), 1026 (P-O-C), 752 (P-C).
**b)** 30-Diethoxyphosphoryl-3-*O*-(3,3'-dimethylglutaryl)betulinic acid **1A** [R = R₁ = Et, R₂ = HOOCCH₂C(CH₃)₂CH₂C(O)O, R₃ = COOH]
   Yield: 27%. M.p. 129-131°C.
   TLC (methylene chloride/ethanol, 15:1, v/v): R_{f} = 0.22.
   Spectroscopic data of the product obtained:
      ¹H NMR (CDCl₃) δ (ppm): 0.70 (m, 1H, H5); 0.73 (s, 3H, CH₃); 0.78 (s, 3H, CH₃); 0.79 (s, 3H, CH₃); 0.87 (s, 3H, CH₃); 0.90 (s, 3H, CH₃); 1.02 (s, 3H, CH₃); 1.10 (s, 3H, CH₃); 1.1 - 2.3 (m, 29H, CH, CH₂); 1.27 (m, 6H, 2 x OCH₂CH₃); 2.31 (m, 2H, CH₂); 2.43 (m, 2H, CH₂); 2.68 (m, 2H, H30); 2.95 (m, 1H, H19); 4.05 (m, 4H, 2 x OCH₂CH₃); 4.43 (m, 1H, H3); 4.93 (m, 1H, H29); 4.98 (m, 1H, H29).
      ¹³C NMR (CDCl₃) δ (ppm): 14.6; 16.3; 16.6; 16.8; 16.9; 18.3; 21.0; 23.9; 26.2; 28.3; 28.5; 28.8; 29.7; 31.0; 32.1; 34.1; 32.4; 34.0; 36.6; 37.2; 37.8; 38.0; 38.1; 40.6; 42.3; 44.1; 45.0; 49.8; 50.5; 55.1; 56.5; 58.6; 62.3; 68.3; 81.0; 113.0; 172.1; 177.5; 182.0.
      ³¹P NMR (CDCl₃) δ (ppm): 28.0.
      IR (KBr) v (cm⁻¹): 1701, 1728 (C=O), 1221 (P=O), 1028 (P-O-C), 789 (P-C).
**c)** 30-Diethoxyphosphoryl-3-*O*-(4,4'-dimethylglutaryl)betulinic acid **1A** [R = R₁ = Et, R₂ = HOOCC(CH₃)₂CH₂CH₂C(O)O, R₃ = COOH]
   Yield: 26%. M.p. 119-124°C.
   TLC (chloroform/ethanol, 15:1, v/v): R_{f} = 0.28.
   Spectroscopic data of the product obtained:
      ¹H NMR (CDCl₃) δ (ppm): 0.75 (m, 1H, H5); 0.72 (s, 3H, CH₃); 0.75 (s, 3H, CH₃); 0.77 (s, 3H, CH₃); 0.83 (s, 3H, CH₃); 0.90 (s, 3H, CH₃); 1.13 (s, 3H, CH₃); 1.15 (s, 3H, CH₃); 1.2 - 2.3 (m, 29H, CH, CH₂); 1.25 (m, 6H, 2 x OCH₂CH₃); 2.5 (m, 2H, H30); 2.93 (m, 1H, H19); 4.04 (m, 4H, 2 x OCH₂CH₃); 4.90 (m, 1H, H3); 4.93 (m, 1H, H29); 4.98 (m, 1H, H29).
      ¹³C NMR (CDCl₃) δ (ppm): 14.7; 16.0; 16.1; 16.4; 16.8; 18.2; 21.0; 23.7; 24.3; 24.7; 26.3; 28.8; 28.2; 29.7; 30.7; 31.0; 32.1; 34.1; 35.1; 36.5; 37.1; 37.9; 38.1; 38.3; 40.6; 40.7; 41.6; 42.3; 50.1; 50.7; 55.2; 56.4; 61.9; 62.0; 80.8; 113.0; 145.0; 177.0; 181.9; 183.1.
      ³¹P NMR (CDCl₃) δ (ppm): 28.0
      IR (KBr) v (cm⁻¹): 1705 (C=O), 1219 (P=O), 1026 (P-O-C), 751 (P-C).
**d)** 29-Diethoxyphosphoryl-3-*O*-(3,3'-dimethylsuccinyl)betulinic acid **1B** [R = R₁ = Et, R₂ = HOOCC(CH₃)₂CH₂C(O)O, R₃ = COOH]
   Yield: 22%. M.p. 150-154°C.
   TLC (chloroform/ethanol, 15:1, v/v): R_{f} = 0.13.
   Spectroscopic data of the product obtained:
      ¹H NMR (CDCl₃) δ (ppm): 0.73 (s, 3H, CH₃); 0.77 (s, 3H, CH₃); 0.79 (s, 3H, CH₃); 0.87 (s, 3H, CH₃); 0.89 (s, 3H, CH₃); 1.20 (s, 3H, CH₃); 1.23 (s, 3H, CH₃); 1.25 (m, 6H, 2 x OCH₂CH₃); 1.98 (br. s, 3H, CH₃); 1.2 - 2.3 (m, 26H, CH, CH₂); 2.40 (d, 1H, *J* = 15.6 Hz, CH); 2.80 (d, 1H, *J* = 15.6 Hz, CH); 3.04 (m, 1H, H19); 3.97 (m, 4H, 2 x OCH₂CH₃); 4.47 (m, 1H, H3); 5.39 (m, 1H, H29).
      ¹³C NMR (CDCl₃) δ (ppm): 14.7; 16.3; 16.4; 16.5; 16.9; 17.3; 18.4; 23.6; 24.3; 25.7; 26.7; 26.8; 28.0; 28.4; 29.7; 29.8; 30.6; 30.9; 33.8; 33.9; 37.1; 37.2; 37.9; 38.2; 40.5; 40.7; 42.2; 42.3; 45.5; 49.5; 49.6; 50.1; 55.1; 56.6; 61.3; 81.3; 111.3; 170.5; 182.0; 183.0.
      ³¹P NMR (CDCl₃) δ (ppm): 18.6.
      IR (KBr) v (cm⁻¹): 1728 (C=O), 1219 (P=O), 1024 (P-O-C), 752 (P-C).
**e)** 29-Diethoxyphosphoryl-3-*O*-(3,3'-dimethylglutaryl)betulinic acid **1B** [R = R₁ = Et, R₂ = HOOCCH₂C(CH₃)₂CH₂C(O)O, R₃ = COOH]
   Yield: 41%. M.p. 131-133°C.
   TLC (methylene chloride/ethanol, 15:1, v/v): R_{f} = 0.12.
   Spectroscopic data of the product obtained:
      ¹H NMR (CDCl₃) δ (ppm): 0.72 (m, 1H, H5); 0.73 (s, 3H, CH₃); 0.78 (s, 3H, CH₃); 0.79 (s, 3H, CH₃); 0.87 (s, 3H, CH₃); 0.89 (s, 3H, CH₃); 1.03 (s, 3H, CH₃); 1.10 (s, 3H, CH₃); 1.25 (m, 6H, 2 x OCH₂CH₃); 1.98 (s, 3H, CH₃); 0.9 - 2.5 (m, 26H, CH, CH₂); 3.0 (m, 1H, H19); 3.98 (m, 4H, 2 x OCH₂CH₃); 4.43 (m, 1H, H3); 5.39 (d, 1H, *J*_{H-P} = 19.2 Hz, H29).
      ¹³C NMR (CDCl₃) δ (ppm): 14.6; 16.0; 16.1; 16.2; 16.4; 18.2; 20.8; 23.6; 24.5; 24.8; 25.0; 25.9; 28.1; 29.6; 29.8; 30.0; 30.7; 32.0; 33.9; 34.1; 35.0; 37.0; 37.1; 37.9; 38.2; 38.7; 40.7; 41.2; 41.5; 41.6; 42.3; 50.1; 55.3; 56.4; 61.4; 80.8; 111.8; 173.0; 181.7; 183.3.
      ³¹P NMR (CDCl₃) δ (ppm): 18.7
      IR (KBr) v (cm-1): 1716 (C=O), 1220 (P=O), 1028 (P-O-C), 754 (P-C).
**f)** 29-Diethoxyphosphoryl-3-*O*-(4,4'-dimethylglutaryl)betulinic acid **1B** [R = R₁ = Et, R₂ = HOOCC(CH₃)₂CH₂CH₂C(O)O, R₃ = COOH]
   Yield: 32%. M.p. 141-146°C.
   TLC (chloroform/ethanol, 15:1, v/v): R_{f} = 0.30.
   Spectroscopic data of the product obtained:
      ¹H NMR (CDCl₃) δ (ppm): 0.7 (m, 1H, H5); 0.84 (s, 3H, CH₃); 0.86 (s, 6H, 2 x CH₃); 0.92 (s, 3H, CH₃); 0.98 (s, 3H, CH₃); 1.23 (s, 3H, CH₃); 1.27 (s, 3H, CH₃); 1.33 (m, 6H, 2 x OCH₂CH₃); 2.07 (s, 3H, CH₃); 1.2 - 2.5 (m, 29H, CH, CH₂); 3.1 (m, 1H, H19); 4.06 (m, 4H, 2 x OCH₂CH₃); 4.49 (m, 1H, H3); 5.48 (d, 1H, *J*_{H-P} = 18.6 Hz, H29).
      ¹³C NMR (CDCl₃) δ (ppm): 14.6; 16.0; 16.1; 16.2; 16.4; 18.2; 20.8; 23.6; 24.5; 24.8; 25.0; 25.9; 28.1; 29.6; 29.8; 30.0; 30.7; 32.0; 33.9; 34.1; 35.0; 37.0; 37.1; 37.9; 38.2; 38.7; 40.7; 41.2; 41.5; 41.6; 42.3; 50.1; 55.3; 56.4; 61.4; 80.8; 111.8; 173.0; 181.7; 183.3.
      ³¹P NMR (CDCl₃) δ (ppm): 18.7.
      IR (KBr) v (cm⁻¹): 1716 (C=O), 1220 (P=O), 1028 (P-O-C), 754 (P-C).

### Example 6.

Preparation of a pharmaceutical based on derivatives according to the invention and intended for oral administration.

The oral pharmaceutical preparation is prepared in the form of tablets with standard sugar coatings based on sucrose or on polyvinyl alcohol, and in the form of hard gelatine capsules, containing from 100 to 400 mg of active ingredient in the form of a phosphonate derivative of 3-carboxyacylbetulinic acid of formula **1A** or **1B,** and containing excipients, such as: cellulose, corn starch, magnesium stearate, silicon dioxide, hydroxypropylmethylcellulose (HPMC), sodium croscarmellose.

### Evaluation of the HIV replication inhibiting ability of the compounds studied

Biological testing of compounds is based on the determination of their cytotoxicity, including determination of CC₅₀ and CC₉₀ (Cytotoxity Concentration) and the assessment of the ability to inhibit viral replication in cell cultures and the determination of IC₅₀ and IC₉₀ (Inhibitory Concentration).
Cytotoxicity of compounds is assessed in cultures of established lymphoid CEMT4 cell lines using the MTT method. The results of viability determination are related to control cultures (100% viability) run in a medium that does not contain the substances studied. The cultures are run in triplicate for every concentration of the tested compounds.
HIV replication inhibition by the studied compounds is assessed in cultures of established lymphoid CEMT4 cell lines. Cells are incubated for 24 hours in a medium (RPMI 10% FBS) supplemented with known concentrations of the tested preparations. The cells are then infected with a laboratory isolate of HIV-1 (wild type, S1). The cultures are prepared in triplicate for every concentration of the tested compound. Cultures containing identical number of infected cells grown in a medium not containing the tested substances are used as a positive control (100% viral replication).
HIV inhibiting ability is determined after 7 days of growing the culture by determining the viral p24 protein in the medium above the cells. The amount of the protein corresponds to the number of newly synthesized viruses. Inhibition of replication is measured by reducing the amount of p24 protein in cultures enriched in test compounds with respect to the amount of p24 in a culture carried out in a standard medium. The amount of p24 viral protein is determined using an anti-p24 immunoenzymatic test. The results are presented in the form of CC₅₀, IC₅₀ and TI (Therapeutic Index) values.

**Table 1. Results of determination of anti-HIV activity of synthesized compounds and of the reference compound, AZT**

| Item | Tested compound | | | Activity | | |
|---|---|---|---|---|---|---|
| | R₂ | | R₃ | CC₅₀ [µM] | IC₅₀ [µM] | TI |
| 1 | HO (Betulin) | | CH₂OH | 46 | NO | - |
| 2 | HO (Betulinic acid) | | COOH | 5 | NO | - |
| 3 | HOOCC(CH₃)₂CH₂COO [3-*O*-(3,3'-dimethylsuccinyl)betulinic acid] | | COOH | 29 | 0.03 | 966 |
| 4 | **1A** | HO | COOH | >42 | >10 | 4.2 |
| 5 | | HOOCC(CH₃)₂CH₂COO | COOH | >69 | 0.02 | >3450 |
| 6 | | HOOCC(CH₃)₂CH₂CH₂COO | COOH | >68 | 0.9 | >75 |
| 7 | | HOOC CH₂C(CH₃)₂CH₂COO | COOH | >68 | 1 | >68 |
| 8 | **1B** | HO | COOH | 80 | >10 | 8 |
| 9 | | HOOCC(CH₃)₂CH₂COO | COOH | >69 | 2 | >34.5 |
| 10 | | HOOCC(CH₃)₂CH₂CH₂COO | COOH | >68 | >10 | 6.8 |
| 11 | | HOOC CH₂C(CH₃)₂CH₂COO | COOH | >68 | 8.8 | >7.73 |
| 12 | AZT | | | >280 | 0.005 | >56000 |

The obtained results of testing phosphonate derivatives of 3-carboxyacylbetulinic acid of formulas 1A and 1B indicate that the most active compound is the derivative 1A (where R = R₁ = Et, R₂ = HOOCC(CH₃)₂CH₂COO, R₃ = COOH) the activity of which is comparable to that of the known 3-*O*-(3',3'-dimethylsuccinyl)betulinic acid, whereas the selectivity of this derivative is higher. Among the derivatives of formula 1B the most active is the compound, where R = R₁ = Et, R₂ = HOOCC(CH₃)₂CH₂COO, R₃ = COOH.

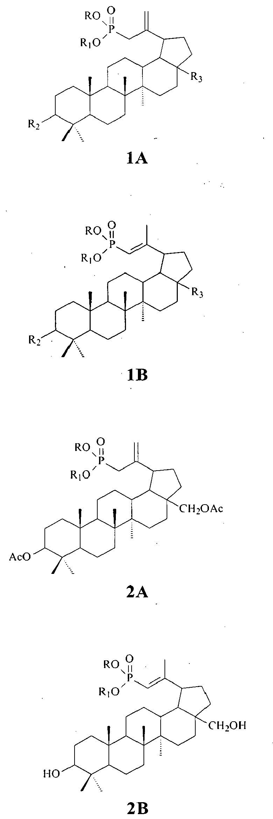

## Claims

1. Phosphonate derivatives of 3-carboxyacylbetulinic acid of antiviral activity, of formula **1A** or of formula **1B,** wherein:
R is an alkyl group (C₁-C₄),
R₁ is an alkyl group (C₁-C₄),
R₂ is a hydroxyl or carbonyl or acetyloxy or carboxyacyloxy group,
R₃ is an acetyloxymethyl or hydroxymethyl or carboxyl group.

2. A method for the preparation of 30-phosphono derivatives of 3-carboxyacylbetulinic acid with antiviral activity, of formula **1A,** wherein:
R is an alkyl group (C₁-C₄),
R₁ is an alkyl group (C₁-C₄),
R₂ is a hydroxyl or carbonyl or acetyloxy or carboxyacyloxy group,
R₃ is an acetyloxymethyl or hydroxymethyl or carboxyl group,
**characterized in that** the method consists of the following steps:
**(a)** compound of formula **2A,** wherein:
R is an alkyl group (C₁-C₄),
R₁ is an alkyl group (C₁-C₄),
is subjected to alkaline hydrolysis in the presence of an aqueous solution of a metal hydroxide selected from KOH and NaOH, with a concentration of 2-4 N, in an organic solvent or in a mixture of organic solvents, wherein the reaction is carried out at room temperature for 0.5 to 4 hours, resulting in 3-acetylbetulin 30-phosphonate,
**(b)** the product of step (a) is reacted with the Jones reagent taken in an amount of 1 to 4 ml per 1 mmol of the product of step (a), in at least 10 ml of an organic solvent per 1 mmol of the product of step (a), resulting in 30-phosphonate of 3-acetylbetulinic acid,
**(c)** the product of step (b) is subjected to alkaline hydrolysis in the presence of an aqueous solution of a metal hydroxide selected from KOH and NaOH, with a concentration of 2-4 N, or an aqueous solution of potassium carbonate K₂CO₃ with a concentration of 1-2 M, in an organic solvent, wherein the reaction is carried out at room temperature for 3 to 24 hours, resulting in 30-phosphonate of betulinic acid,
**(d)** the product of step (c) is reacted with a dicarboxylic acid anhydride or with a dicarboxylic acid, at a molar ratio of between 1:1 and 1:10, for at least 30 minutes.

3. Method according to claim 2, **characterized in that** the reaction of step (a) is carried out in a solvent selected from methanol and ethanol, or in a mixture of tetrahydrofuran and methanol (1:3 to 3:1, v/v), or in a mixture of methanol and chloroform (preferably 1:1, v/v).

4. Method according to claim 2, **characterized in that** step (b), that is the reaction of the product of step (a) with the Jones reagent, is carried out in an acidic medium at a temperature of from -10°C to room temperature, most preferably at a temperature of 20 to 24°C.

5. Method according to claim 2, **characterized in that** the organic solvent used in step (b) is acetone or dichloromethane or a mixture thereof, most preferably at the solvents ratio of 1:1, v/v.

6. Method according to claim 2, **characterized in that** the reaction of step (c) is carried out in a solvent selected from methanol and ethanol, or in a mixture of tetrahydrofuran and methanol (1:3 to 3:1, v/v).

7. Method according to claim 2, **characterized in that** step (d), that is the reaction of the product of step (c) with a dicarboxylic acid anhydride or with a dicarboxylic acid, is carried out in an organic solvent in an amount necessary to dissolve the product of step (c), in the presence of 4-dimethylaminopyridine at a molar ratio of 1:1 to 1:2 based on the product of step (c), preferably in a microwave reactor, preferably at a temperature of 120 to 160°C.

8. Method according to claim 7 **characterized in that** the organic solvent used is one selected from the group consisting of: pyridine, methylene chloride, chloroform, toluene, diethyl ether, dimethylsulfoxide, acetonitrile, tetrahydrofuran and dioxane.

9. A method for the preparation of 29-phosphono derivatives of 3-carboxyacylbetulinic acid with antiviral activity, of formula **1B,** wherein:
R is an alkyl group (C₁-C₄),
R₁ is an alkyl group (C₁-C₄),
R₂ is a hydroxyl or carbonyl or acetyloxy or carboxyacyloxy group,
R₃ is an acetyloxymethyl or hydroxymethyl or carboxyl group,
**characterized in that** the method consists of the following steps:
**(a)** compound of formula **2B,** wherein:
R is an alkyl group (C₁-C₄),
R₁ is an alkyl group (C₁-C₄),
is reacted with the Jones reagent taken in an amount of 2 to 6 ml per 1 mmol of the compound of formula **2B,** in at least 10 ml of an organic solvent per 1 mmol of the compound of formula **2B,** resulting in 29-phosphonate of betulonic acid,
**(b)** the product of step (a) is reduced by sodium or lithium borohydride, resulting in 29-phosphonate of betulinic acid,
**(c)** the product of step (b) is reacted with a dicarboxylic acid anhydride or with a dicarboxylic acid, at a molar ratio of between 1:2.5 to 1:10, for at least 30 minutes.

10. Method according to claim 9, **characterized in that** step (a), that is the reaction of the compound of formula 2B with the Jones reagent, is carried out in an acidic medium at a temperature of from -10°C to room temperature, most preferably at a temperature of 20 to 24°C.

11. Method according to claim 9, **characterized in that** the organic solvent used in step (a) is acetone or dichloromethane or a mixture thereof, most preferably at the solvents ratio of 1:1, v/v.

12. Method according to claim 9, **characterized in that** step (b), that is the reaction of the product of step (a) with sodium or lithium borohydride at a molar ratio of at least 1:1.3 is carried out in an organic solvent at a ratio of at least 10 ml of the solvent per 1 mmol of the product of step (a), at room temperature, for at least 30 minutes.

13. Method according to claim 12 **characterized in that** the organic solvent used is one selected from the group consisting of: tetrahydrofuran, diethyl ether, isopropanol, dioxane, ethanol and methanol.

14. Method according to claim 9, **characterized in that** step (c), that is the reaction of the product of step (b) with a dicarboxylic acid anhydride or with a dicarboxylic acid, is carried out in an organic solvent in an amount necessary to dissolve the product of step (b), in the presence of 4-dimethylaminopyridine at a molar ratio of 1:1 to 1:2 based on the product of step (b), preferably in a microwave reactor, preferably at a temperature of 120 to 160°C.

15. Method according to claim 14 **characterized in that** the organic solvent used is one selected from the group consisting of: pyridine, methylene chloride, chloroform, toluene, diethyl ether, dimethylsulfoxide, acetonitrile, tetrahydrofuran and dioxane.

16. The use of phosphonate derivatives of 3-carboxyacylbetulinic acid with antiviral activity, of formula **1A** or **1B,** wherein:
R is an alkyl group (C₁-C₄),
R₁ is an alkyl group (C₁-C₄),
R₂ is a hydroxyl or carbonyl or acetyloxy or carboxyacyloxy group,
R₃ is an acetyloxymethyl or hydroxymethyl or carboxyl group,
in the manufacture of pharmaceutical agents for suppressing HIV-1 replication.
